# EUROPEAN PATENT APPLICATION

(11) **EP 3 556 359 A1**
(43) Date of publication of application: **23.10.2019**
(21) Application number: 16917014.9
(22) Date of filing: 19.12.2016
(51) Int. Cl.: A61K 9/70

(54) **ENERGISING PATCH FOR SPORTSPEOPLE**

(71) Applicant: Nutritape, S.L., 43830 Torredembarra, Tarragona (ES)
(72) Inventor: HABA CARDÓ, José María, 43830 Torredembarra (Tarragona) (ES)
(86) International application number: PCT/ES2016/070910
(87) International publication number: WO 2018/115535

(57) **Abstract**

Transdermal patch consisting of:
a.-) a membrane of a flexible polymeric material acting as a support layer, forming the outer cover of the patch, to which the following element is adhered using a copolymer adhesive:
b.-) a layer of non-woven fabric (or TNT) comprising:
i. Aminoacids L-Leucine, L-Isoleucine and L-Valine, and vitamins B1 and B5 as active ingredients; or their chemically acceptable salts or byproducts;
ii. excipients selected from the group consisting of modulators for the release of active ingredients, optimisers for the absorption of active agents, solvents, surfactants and emulsifiers, and

c.-) a transparent removable plastic film covering the above elements and the adhesive cord from environmental conditions. Said film must be removed by the athlete before adhering the patch to his skin.

## Description

### TECHNICAL FIELD

The present invention relates to the field of nutrition, in particular to the field of nutritional supplements or food for athletes. This is not a food substitute.

Transdermal technology was born in the USA in December 1979. Since then, it has been applied to medicine, pharmacy and in the last decade to cosmetics. Positive results in all these fields guarantee transdermal technology. Therefore, we find pain reliever patches, anti-smoking, for the heart, contraceptives, anti-wrinkle, anti-mosquitos, for scars, anti-ageing, etc.

The present invention applies this technology to sports nutrition for the first time. This breaks away from the paradigm of traditional nutrition (the intake of nutrients for athletes) and the application of patches in the aforementioned fields (medicine, pharmacy and cosmetics) to focus on improving the performance of an athlete devoted to more than one hour of continuous physical activity (for example: cycling, marathon, triathlon, skiing, mountaineering, etc.).

The motivation behind this new application is the discomfort suffered by athletes during the digestive process. They, in a conventional manner, carry out the oral ingestion of nutrients needed during the physical activity using solid formulas such as energy bars, energy gels, energy goodies, energy drinks, tablets, capsules, granules; and liquid formulas such as syrups, mouthwashes or similar.

Thus, the following limitations and problems are detected in the current methodology of energy supply for athletes:
1. Up to 70% of what they eat is destroyed during the digestive process (primarily by stomach acids and intestinal transit).
2. The digestive process lasts between 30 minutes and 3 hours, depending on the format of the product (energy gel or pasta dish). Therefore, it takes a long time from the moment they ingest a nutrient until it reaches the bloodstream and from there to the muscle to practice the sport at issue. It must be taken into account that athletes need said nutrients to be immediately available.
3. Eating during sports (marathon, triathlon, ultra marathon, cycling, etc.) causes digestive problems as well as gastroenteritis and/or belly pain. Many athletes are forced to leave a competition due to stomach discomfort and/or stomach cramps followed by vomiting, diarrhoea or abdominal pain.
4. Usually, energy products have a high sugar content so that ingesting them causes insulin spikes and a subsequent decrease thereof resulting in low performance.

To overcome these limitations, the inventors have developed a transdermal patch containing the nutrients needed by athletes to develop their physical activity without digestive discomfort. It consists of:
a.-) a membrane of a flexible polymeric material acting as a support layer, forming the outer cover of the patch, to which the following element is adhered using a copolymer adhesive:
   b.-) a layer of non-woven fabric (or TNT) comprising:
      i. Aminoacids L-Leucine, L-Isoleucine and L-Valine, and vitamins B1 and B5 as active ingredients; or their chemically acceptable salts or byproducts;
      ii. excipients selected from the group consisting of modulators for the release of active ingredients, optimisers for the absorption of active agents, solvents, surfactants and emulsifiers, and
   c.-) a transparent removable plastic film covering the above elements and the adhesive cord from environmental conditions. Said film must be removed by the athlete before adhering the patch to his skin.

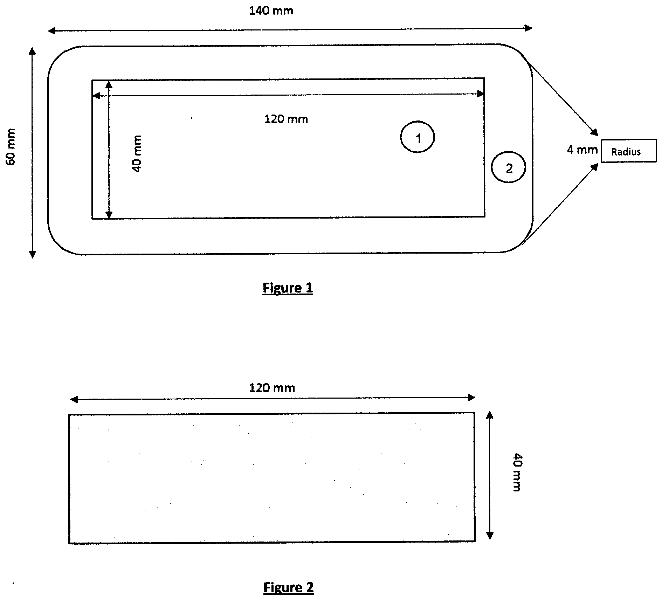

An accurate representation of said patch can be seen in figure 1. Obviously, element c) is not visible for being transparent.

Therefore, the limitations and problems detected are solved:
1. 100% of the nutrients necessary for sports are used.
2. They are supplied from the first moment or from the moment chosen by the athlete. Athletes just have to remove the protective film (layer 3 in figure 1) and adhere the patch to their skin.
3. The transit through the digestive tract and the hepatic first pass are skipped so there will be no digestive discomfort. Once again, this is a nutritional supplement.
4. Absorption is slow and insulin spikes are avoided.

### BACKGROUND INFORMATION AND PRIOR ART

Traditionally, with respect to a method of administration of nutrients or active ingredients, we know that oral administration uses solid formulas such as tablets, capsules, granules, energy bars, energy gels, energy goodies, energy drinks and liquid formulas such as syrups, mouthwashes or similar.

In addition, the oral route presents another series of limitations such as the amount of energy consumed in the digestion process itself, the time until nutrients or active ingredients reach the bloodstream, the insulin spikes produced in the process, discomfort or bad digestion when executing sports and digesting at the same time, etc.

The topical or dermal administration of systemically acting nutrients provided by the inventors of the present invention is innovative. This modern system enables dosage control and the constant, sustained and controlled release of nutrients, in a system designed for application to a specific area of the skin, which serves as a support or vehicle for one or several nutrients intended to have an energising effect after their release and passing through the skin. Therefore, the limitations and problems detected are solved:
1. 100% of the nutrients necessary for sports are used.
2. Supplied from the first moment or from the moment chosen by the athlete. They just have to remove the protective film and adhere the patch to their skin.
3. Transit through the digestive tract and the hepatic first pass are skipped so there will be no discomfort. Once again, this is a nutritional supplement.
4. Absorption is slow, and insulin spikes are avoided.

Thus, the inventors of the present invention have developed a formulation in the form of a transdermal patch to ensure that all the nutrients incorporated therein reach the bloodstream to facilitate athletes administration thereof. For this, they have obtained a patch with multiple advantages such as a reduction in the frequency of administration, improved compliance, ease of dispense and suspension of the treatment, the complete and immediate absorption of nutrients required by athletes (without delaying their activity due to absorption and/or metabolism phenomena). Therefore, by skipping the digestive process and the hepatic first pass, inventors make sure to incorporate into the bloodstream the amount of nutrients needed by the muscles for sporting activities of more than one continuous hour, taking into account that during the digestive process up to 70% of the nutrients ingested are destroyed, resulting in a very low amount to be absorbed by the intestines and from there, to be incorporated into the bloodstream so that the human body can deliver them where they are required.

These aspects are innovative since they improve the well-being and performance of people who perform intense sports (marathon, triathlon, *Iron Man,* cycling, mountaineering, etc.), they need stable levels of nutrients from the beginning of the physical activity. Also, an oral intake of nutrients is not recommended in these sports due to the digestive problems caused.

Documents in the prior art describe compositions in the form of transdermal patches suitable for the release of active agents, such as drugs such as fentanyl (ES2270746T3), estrogens (ES2190472 T3), etc. However, when we try to find prior art documents referring to nutritional supplements, we find very few ones.

For example, we find EP1948221A1 which refers to patch formulations with human growth hormone (HGH). This European patent application describes a formulation in the form of a transdermal patch comprising HGH, at least one sugar, an amino acid or polyol, and a buffer, wherein the buffer maintains the pH of the formulation in the range of about 5 to 9 and the formulation contains neither glycine nor mannitol. However, this patch is for treating a deficit in growth hormone, and it is intended for an extended release, not immediate, so it usually requires a concomitant oral treatment of the hormone.

We also found document TW200906457A regarding a transdermal patch comprising a support layer, an adhesive layer fixed under the support layer, and a layer formed between the support layer and the adhesive layer to provide the improved materials. The composite layer comprises natural water substances, various electrolytes, minerals, vitamins, and chemically enhanced enzymes, which can also be cellular reactivators or chelating detoxification agents. In this patent, they speak of a reservoir in which the chemical or natural components are found, but it is not suitable for athletes or people performing long physical activities of at least 1 hour.

Another document we found is patent ES2230872 which refers to a transdermal patch containing at least one active ingredient that influences the levels of blood lipids. This invention describes a preparation in the form of a transdermal therapeutic system containing, in a self-adhesive layer being coated with an impermeable upper layer to the active principle on its farthest face from the skin, at least one active principle influences the levels of blood lipids and at least one adjuvant promoter of the permeation of the active principle through the skin, characterised by the active principle of the group of active ingredients inhibiting the hydroxymethylglutaryl-CoA reductase being selected, the self-adhesive layer is a paste based on polyacrylate or a silicone-based paste, and said excipient being selected from the group comprising pyrrolidone derivatives, fatty acids, fatty alcohols, fatty acid esters, fatty acid ethers, paraffin derivatives, terpenes, monoalkyl ethers of ethylene glycol, polyoxyethylenated alkyl ethers, aryl ethers, polyoxyethylenates, polyoxyethylenated alkyl esters, polyoxypropylenated alkyl ethers, fatty acid and propylene glycol derivatives, glycerol fatty esters, polysorbates, poloxamers, dialkylsulphoxides, urea and its derivatives, glycerin, natural oils, laurocapramas, phospholipids, a-midas, amino acids, N, N-dimethylformamide, N-methylformamide, acetonides, calcium thioglycolate, propylene glycol, polyethylene glycol, alkyl sulfates, sodium lauryl sulfate, tetrahydrofurfuryl alcohol, macrocyclic compounds or polar solvents such as panthenol. However, this patch contains agents for treating and controlling cholesterol and is intended for an extended release, not immediate.
Taking into account all the background above, we can conclude that none of them discloses the use of patches as a vehicle to supply nutritional supplementation for athletes or people who perform long physical activities of at least 1 hour.

Thus, we can conclude that the transdermal patch object of the present invention is novel since no prior art document defining an immediate release patch of nutritional supplements including the branched-chain amino acids L-Leucine, L-Isoleucine and L-Valine, and B vitamins, in a transdermal patch for sports activities of more than one hour has been found.

### DESCRIPTION OF THE INVENTION

According to the present invention "active agent", "active principle", "nutrient", "food supplement", "nutritional supplement", "dietary supplement", "nutritional supplementation" or "active ingredient" - both in singular as in plural and indistinctly are the ingredients or active components that are part of the patch and that are subject to absorption or administration through the patch object of the invention. That is, everything that is part of the patch that is not excipients or support materials of the same and whose purpose is to provide energy to the athlete by entering the bloodstream.

However, to date, all applications have been aimed at the medical and cosmetic field and have never been developed as a nutritional supplement for elite or amateur athletes who practice high-performance sports with specific needs unresolved by today's proposals in the prior art.

Thus, the enormous technical advantage provided by the present invention to athletes is more than evident: transdermal patch to nourish the muscles of the athlete, without causing digestive discomfort.

For all the above, the researchers of the present invention have developed a transdermal patch with specific nutrients: branched amino acids L-Leucine, L-Isoleucine and L-Valine, and vitamins B1 and B5.

The patch preferably comprises:
a.-) a membrane of a flexible polymeric material acting as a support layer, forming the outer cover of the patch, to which the following element is adhered using a copolymer adhesive:
   b.-) a layer of non-woven fabric (or TNT) comprising:
      i. Aminoacids L-Leucine, L-Isoleucine and L-Valine, and vitamins B1 and B5 as active ingredients; or their chemically acceptable salts or byproducts;
      ii. excipients selected from the group consisting of modulators for the release of active ingredients, optimisers for the absorption of active agents, solvents, surfactants and emulsifiers, and
   c.-) a transparent removable plastic film covering the above elements and the adhesive cord from environmental conditions. Said film must be removed by the athlete before adhering the patch to his skin.

A graphic representation can be found in Figure 1, shown from the surface to be applied on the skin.

The main advantages of the energising patch for athletes include the following:
- Controlled release of nutrients to the muscles.
- Constant and time-sustained plasma levels.
- Elimination of the hepatic first-pass effect, in which a large part of the amino acids and vitamins ingested are destroyed.
- The possibility of instantly stopping administration by just peeling off the skin patch. This is impossible to replicate with ingested food.
- Convenience, because the main supporting body is a membrane of flexible polymeric material.

### MANUFACTURING EXAMPLE

### Example 1: energising patch for athletes

We will describe the process of manufacturing an energising patch for athletes, consisting of a membrane of flexible polymeric material measuring 140 millimetres long by 60 millimetres width and rounded edges (by a circumference of 4 millimetres radius). Said membrane of flexible material will have a layer of non-woven fabric (or TNT) attached to its lower part, which will contain the following nutrients:

**Table 1**

| | | **Tolerance (grams)** | |
|---|---|---|---|
| | **Amount (grams)** | **- 5%** | **+ 5%** |
| L-Leucine | 0.252 | 0.239 | 0.265 |
| L-Isoleucine | 0.126 | 0.120 | 0.132 |
| L-Valine | 0.126 | 0.120 | 0.132 |
| Vitamin B1 (mg) | 0.001 | 0.0010 | 0.0011 |
| Vitamin B5 (mg) | 0.005 | 0.0048 | 0.0053 |

A graphic representation can be seen in figure 1, shown to the reader from its bottom, to see the two bodies composing it:
1) Part 1 is a support or layer of non-woven fabric (or TNT) containing vitamins B1 and B5, and the branched amino acids L-Leucine, L-Isoleucine and L-Valine, according to Table 1 on the previous page. It measures 120 millimetres long and 40 millimetres wide.
2) Part 2 is a membrane made of flexible polymer material with copolymeric acrylic adhesive to keep the patch adhered to the athlete's skin during the physical exercise without the patch bothering or producing tension. It measures 140 millimetres long and 60 millimetres wide.

### PREPARATION OF THE PATCH OBJECT OF THIS PATENT: PART 1

Step 1) In a container at a temperature lower than 28° Celsius, a properly cross-linked non-woven fabric layer (TNT) is introduced. This layer will contain vitamins B1 and B5, amino acids L-Leucine, L-Isoleucine and L-Valine as indicated in Table 1, which will be incorporated in Step 2.

Next, an acrylic copolymer adhesive formulated in such a way that it is able to incorporate the exogenous components inside without damaging the physical-chemical properties of the copolymer is added. In the case of this patent, these exogenous components are vitamins B1 and B5 and branched amino acids L-Leucine, L-Isoleucine and L-Valine.

Step 2) Said exogenous substances are placed in a separate container, equipped with a helix mixer, and solvents suitable for contacting the exogenous components are added without altering them physically or chemically. Through this step, the exogenous substances are mixed with the solvents by rotating the helix very slowly until they are completely mixed and dissolved.

Step 3) The homogeneous mixture obtained in Step 2 is inserted in Step 1 using an immersion stirrer. We obtain the non-woven fabric (TNT) with the exogenous components, the adhesive (acrylic copolymer) and the solvents.

Step 4) With a rotary machine, the TNT fabric obtained in Step 3 is applied on a continuous sheet of silicone coated paper 75 microns thick, in order to cover the adhesive and the exogenous components, to protect them from environmental conditions, preserving its adhesive and functional properties. Then at a speed of 4-6 meters per minute, they pass through 4 furnace stations, in which the first furnace is set at a temperature of 40° C, the second at 50° C, the third at 60° C and the fourth one at 90° C.

When exiting the furnaces, the adhesive layer is completely free of solvents, which evaporated in the furnace stations. The thickness of the adhesive is around 250 gr/m2.

The result is that the adhesive layer, protected on its upper part by the silicone sheet, adheres on its lower part to the TNT fabric (non-woven fabric), forming a set of 3 bodies: silicone coated paper, adhesive and TNT fabric that includes the exogenous substances in the amounts specified in Table 1.

Subsequently, this set of 3 bodies obtained in the form of a coil is cut into rectangles using a specific die, measuring 40 millimetres wide and 12 millimetres long.

Therefore, part 1 of the patch containing the exogenous part (TNT with branched amino acids and vitamins B) of figure 1 is obtained. It is represented in figure 2, shown from its upper part.

### PREPARATION OF THE PATCH OBJECT OF THIS PATENT: PART 2

In a container, an adhesive is introduced at a temperature lower than 28° Celsius, preferably an acrylic copolymer with sufficiently high adhesive properties. Specifically, these are: 30 g/m² of dry PET of 50 microns at 180° Peel, N/25 mm in steel ≥ 25, both measured in a loop Tack on steel N ≥ 12. This is added solvents that allow the adhesive to be easy to manage and apply with industrial machinery.

With a rotating machine, this adhesive is applied on a membrane made of flexible polymer material (for example, high elasticity polyurethane foam). It is then covered with silicone coated paper to preserve it from environmental conditions and thus preserve its adhesive properties.

This set formed by a sheet of flexible polymeric material, acrylic copolymer adhesive with sufficiently high adhesive properties, solvents and silicone coated paper, passes at a speed of 11 meters per minute through 4 furnace stations, in which the first furnace is set at a temperature of 50° C, the second at 70° C, the third at 90° C and the fourth one at 120° C.

When exiting the furnaces, the adhesive layer is completely free of solvents, which evaporated in the furnace stations; the thickness of the adhesive is around 50 gr/m2.

When exiting the furnaces, the silicone coated paper is coupled to a membrane of flexible polymer material, protecting the acrylic copolymer from the air and environmental elements.

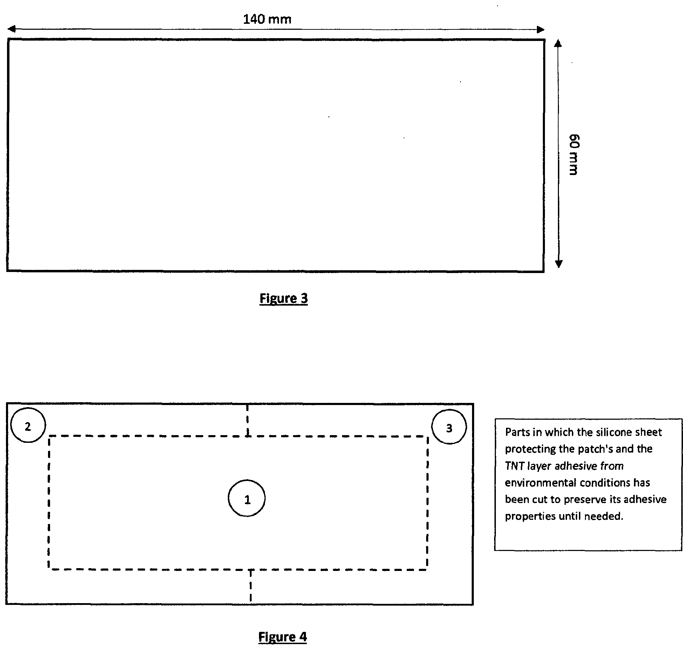

At this point, a rotary machine drilling die cuts a rectangle 6 centimetres wide by 14 cm long, as shown in Figure 3.

The patch obtained this way already has enough stickiness to allow prolonged exposure to the athlete's skin, keeping the layer of non-woven fabric or TNT with the active exogens in contact with the dermis (PART 1).

### FINAL PRODUCT ASSEMBLY

### a) Manual process:

A patch manufactured in Part 2 is taken and placed on a flat surface on the side of the flexible material membrane, leaving the lower part visible to the operator. That is, the part with the silicone coated paper protecting the adhesive. The central part of the silicone-coated paper, which corresponds exactly to the rectangle of the same measurements of the TNT fabric obtained in Part 1, is removed with a cutting element, and the non-woven fabric (TNT) containing the exogenous components is glued.

The operator then, makes two cuts in the silicone-coated paper furthest from the non-woven fabric layer (or TNT), to allow the user of said patch to remove said protective layers with ease.

By doing this, an adhesive edge around 1 cm long is left on the membrane of the flexible material, which will be the adhesive of sufficiently high adhesive properties in contact with the athlete's skin, holding the patch during the entire duration of the sporting activity.

An outline of the manual cuts is shown in Figure 4.

### b) Automated procedure:

Once the TNT fabrics are bonded to the membrane of flexible polymer material, and stacked, a die or cutting tool gives them the final size and shape. Specifically, 60 millimetres wide, 140 millimetres long and rounded edges (4 millimetres radius).

The energising patch for athletes is already finished and ready for use. A graphic representation of it can be seen in Figure 1, shown from its bottom to see the elements comprising it.

With this industrial process, we obtain the whole object of this patent, which contains the active principles useful for practising sports. To apply it on the skin athletes will just have to remove the three parts in which the silicone-coated paper has been divided to reveal the adhesive, allowing said set to remain attached during the whole sporting activity.

We want to highlight the manual assembly of the TNT fabric with exogenous components on the membrane of flexible polymeric material to indicate that:
- the novelty requirement is widely justified, as this is the first time that nutrients are supplied to the muscles through the dermal route, instead of ingesting them as currently done.
- the inventive step requirement is more than fulfilled, by patenting this energising patch for athletes. Instead of ingesting the nutrients, they are delivered to the athlete by the dermal route. According to laboratory tests carried out, the absorption of these nutrients is faster, not causing digestive or intestinal discomfort, and supply is interrupted by removing the patch from the skin. The latter is not possible with the current ingestion method. In our opinion, the improvement in safeness offered by our invention is more than evident.
- finally, the industrial application requirement is widely fulfilled, since the patch object of this patent is machine-aided manufactured in two parts, and only the bonding of both parts is manual.

### BIBLIOGRAPHY SUPPORTING THE INCLUSION OF CERTAIN NUTRIENTS IN THIS ENERGISING PATCH

The inventor considers that providing a bibliography that supports the need for athletes to avoid food intake during sporting activities is of relevant importance:
Regarding the discomfort caused by food intake during sporting activities: "Gastrointestinal Complaints During Exercise: Prevalence, Etiology, and Nutritional Recommendations", by Erick Prado de Oliveira, Roberto Carlos Burini, and Asker Jeukendrup. Published in the "Sports Medicine" magazine 2014, under number DOI 10.1007/s40279-014-0153-2., Pages 79 to S85.

It analyses the effect of poor nutritional planning before and during the race performed by endurance athletes. It concludes that around 30 and 50% suffer from gastrointestinal problems during the race, preventing them from improving times, properly recovering from the race, and enjoying their favourite sport.

The case of long-distance runners is mentioned. Cases of nausea, vomiting, abdominal pain and diarrhoea in athletes who participate in races between 67 and 161 kilometres is very common. It is estimated that between 37% and 89% of them suffer from these conditions.

And concerning specific food for endurance athletes, "Food, Nutrition and Hydration in Sports", published in Madrid, March 2009, by the Service of Medicine, Endocrinology and Nutrition of the Centre for Sports Medicine, of the National Sports Council, Number NIPO: 663-09-051-X, Legal Deposit: M-14321-2009, by Dr Nieves Palacios Gil-Antuñano, Dr Zigor Montalvo Zenarruzabeitia and Mrs Ana Maria Ribas Camacho.

But these nutrients put together and duly proportioned between them will further increase athletic performance. And this is the fundamental purpose of this patent: to unite application technology, nutrients, and amount of nutrients, to market a product that substantially improves sports performance. This is borne out by laboratory tests.

## Claims

1. Transdermal patch consisting of three layers arranged one above the other in a solid manner where there is a first outer layer that is a waterproof coating sheet that acts as a support for the others, an intermediate layer that is a reservoir of active principle or nutrient called "Release module", and an adhesive layer or fastening system on the skin.

2. Transdermal patch according to claim 1, **characterised by** a support layer made of synthetic textile fibre.

3. Transdermal patch according to claim 1, **characterised by** a support layer made of flexible polymeric material.

4. Transdermal patch according to the preceding claims, **characterised by** additionally comprising a fourth reservoir layer.

5. Transdermal patch according to the preceding claims, **characterised by** additionally comprising a detachable plastic sheet that must be removed before application.

6. Transdermal patch according to claim 1, **characterised by** a layered transdermal patch type where the "release module" is selected from the group consisting of coated polymer membranes, hydrogels, elastomeric layers or adhesive layers.

7. Transdermal patch according to claim 4, **characterised by** being a reservoir-type transdermal patch.

8. Use of the transdermal patch of previous claims as a vehicle for nutritional supplementation of people with nutritional deficits.

9. Use of the transdermal patch of previous claims as a vehicle for nutritional supplementation of people who make a physical effort of more than one hour continued.

10. Use of the transdermal patch of previous claims as a vehicle for nutritional supplementation of athletes.

11. Use of the transdermal patch according to claim 10 as a vehicle for nutritional supplementation in selected activities of the group consisting of marathon, triathlon, ironman, cycling, running and trail running.

12. Transdermal patch according to the preceding claims, **characterised by** comprising at least the branched amino acids L-Leucine, L-Isoleucine and L-Valine as active ingredients.

13. Transdermal patch according to the preceding claims, **characterised by** comprising a ratio between the 3 branched amino acids L-Leucine, L-Isoleucine and L-Valine of 2: 1: 1. That is, same amount of L-Isoleucine and L-Valine, and twice as much L-Leucine.

14. Transdermal patch according to the preceding claims, **characterised by** comprising a ratio between the 3 branched amino acids L-Leucine, L-Isoleucine and L-Valine of 4: 1: 1. That is, the same amount of L-Isoleucine and L-Valine, and fourfold of L-Leucine.

15. Transdermal patch according to the preceding claims, **characterised by** comprising a ratio between the 3 branched amino acids L-Leucine, L-Isoleucine and L-Valine of 8: 1: 1. That is, the same amount of L-Isoleucine and L-Valine, and eightfold of L-Leucine.

16. Transdermal patch according to the previous claims **characterised by** comprising the branched amino acids L-Leucine, L-Isoleucine and L-Valine, and vitamins B as active ingredients.

17. Transdermal patch according to the previous claims **characterised by** comprising the branched amino acids L-Leucine, L-Isoleucine and L-Valine, and sodium and potassium salts as active ingredients.

18. Transdermal patch according to the previous claims **characterised by** comprising the branched amino acids L-Leucine, L-Isoleucine and L-Valine, vitamins B, and sodium and potassium salts as active ingredients.

19. Transdermal patch according to the preceding claims, **characterised by** comprising a content by weight of the following active ingredients selectable between the following ranges:
• L-Leucine: 0.1 grams to 40 grams
• L-Isoleucine: 0.1 grams to 5 grams
• L-Valine: 0.1 grams to 5 grams
• B vitamins: 0.1 milligrams to 40 milligrams
• Sodium salts: 0.1 milligrams to 40 milligrams
• Potassium salts: 0.1 milligrams to 40 milligrams

20. Transdermal patch consisting of:
a). a membrane of a flexible polymeric material acting as a support layer, forming the outer cover of the patch, to which the following element is adhered using a copolymer adhesive:
b). a layer of non-woven fabric (or TNT) comprising:
i. Aminoacids L-Leucine, L-Isoleucine and L-Valine, and vitamins B1 and B5 as active ingredients; or their chemically acceptable salts or byproducts;
ii. excipients selected from the group consisting of modulators for the release of active ingredients, optimisers for the absorption of active agents, solvents, surfactants and emulsifiers, and
c). a transparent removable plastic film covering the above elements and the adhesive cord from environmental conditions. Said film must be removed by the athlete before adhering the patch to his skin.
